(19) Europäisches Patentamt
European Patent Office
Office européen des brevets

(11) Numéro de publication: **0 276 583**
**A1**

(12) # DEMANDE DE BREVET EUROPEEN

(21) Numéro de dépôt: **87400193.6**

(22) Date de dépôt: **29.01.87**

(51) Int. Cl.⁴: **C07D 233/56**, A61K 31/415

(43) Date de publication de la demande:
**03.08.88 Bulletin 88/31**

(84) Etats contractants désignés:
**AT BE CH DE ES FR GB GR IT LI LU NL SE**

(71) Demandeur: **Instituto de Investigacion Y Deserrollo Quimico Biologico S.A.**
**Luis Cabrera 63**
**E-28002 Madrid(ES)**

(72) Inventeur: **Soria Soria, Asuncion**
**Luis Cabrera 63**
**E-28002 Madrid(ES)**
Inventeur: **Galiano Ramos, Joaquin Alvaro**
**Tebas no 23**
**Las Rozas Madrid(ES)**

(74) Mandataire: **Gallochat, Alain**
**SOCIETE D'ETUDES SCIENTIFIQUES ET INDUSTRIELLES DE L'ILE DE FRANCE 46,**
**Boulevard de Latour Maubourg**
**F-75340 Paris Cédex 07(FR)**

(54) **Nouveaux dérivés d'imidazole, leurs procédés de préparation et leurs propriétés antifongiques.**

(57) La présente invention concerne de nouveaux dérivés d'imidazole de formule :

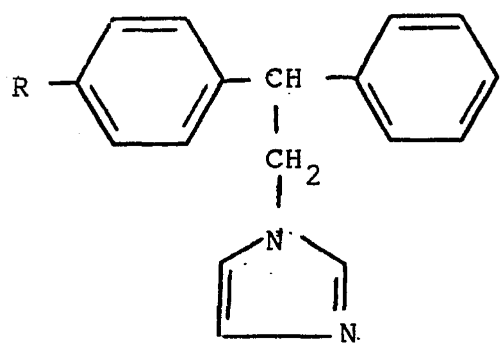

dans laquelle R représente un atome d'halogène, ainsi que leurs sels d'addition pharmaceutiquement acceptables.
Les composés de l'invention sont utiles comme antifongiques.

EP 0 276 583 A1

## NOUVEAUX DERIVES D'IMIDAZOLE, LEURS PROCEDES DE PREPARATION ET LEURS PROPRIETES ANTIFONGIQUES

La présente invention concerne de nouveaux dérivés d'imidazole doués d'une puissante activité antimicrobienne vis à vis des champignons pathogènes, moisissures, levures et bactéries Gram-positives. Ces composés sont utiles pour le traitement d'infections dermiques et vaginales telles que candidose vaginale, tinea corporis, tinea pedis, tinea cruris, pytiriasis versicolor et autres infections dermiques.

Les composés de l'invention sont chimiquement apparentés à d'autres imidazoles antifongiques tels le clotrimazole et le miconazole mais, leur activité antifongique s'est révélée très supérieure à celle des composés connus, ce qui était tout à fait inattendu.

De plus, certains sels des composés de l'invention sont solubles dans l'eau, ce qui permet de préparer des formes galéniques adaptées à l'application sur les muqueuses, les yeux, les oreilles, etc.

En 1944, Woolley signala que des benzimidazoles avaient une activité antifongique et antibactérienne et nota que les effets biologiques du benzimidazole et de ses dérivés pouvaient être contrecarrés par des purines : l'adénine et la guanine.

Cependant, les mycoses n'étaient pas très répandues à cette époque et cette découverte fut donc ignorée.

En 1952, Jerchel et al. reprirent certaines des observations de Woolley et signalèrent que certains dérivés de benzimidazole substitué présentaient une activité antifongique remarquable. Ceci encouragea d'autres groupes de chercheurs à rechercher dans ce groupe chimique un agent thérapeutique intéressant.

Le chlormidazole, dérivé de chlorobenzylimidazole actif contre de nombreux chamignons et bactéries Gram-positives, a été mis au point et commercialisé sous forme de crème à 5 % à usage topique.

Ce composé fut le premier dérivé d'imidazole commercialisé ayant fait l'objet d'essais cliniques.

Le domaine des imidazoles antifongiques prit de l'ampleur en 1969 lorsque trois composés issus de deux laboratoires différents furent divulgués : le clotrimazole (Bayer A.G), le miconazole et l'econazole (Janssen Pharmaceutica).

La chimiothérapie antifongique était un domaine si nouveau et concurrentiel que le premier rapport concernant la synthèse du clotrimazole ne parut que trois ans après la première publication traitant des propriétés antifongiques de ce composé.

Tous ces dérivés d'imidazole sont maintenant activement lancés sur le marché, ce qui montre le succès de ces premières recherches. Malheureusement, cela prouve aussi la lenteur de l'évolution de la série des dérivés de l'imidazole, au cours des 16 dernières années.

Malgré la lenteur du développement de cette classe de composés, de nombreux dérivés d'imidazole à large spectre d'activité antifongique, ont été préparés et étudiés depuis l'introduction du clotrimazole. Les plus importants des dérivés d'imidazole développés et commercialisés au cours des dernières années sont le kétoconazole (Janssen Pharmaceutica) et le bifonazole (Bayer A.G.).

Les formules des composés les plus importants développés par Janssen (kétoconazole, miconazole, econazole) et par Bayer (clotrimazole, bifonazole) sont représentées par les figures 1 et 2 respectivement.

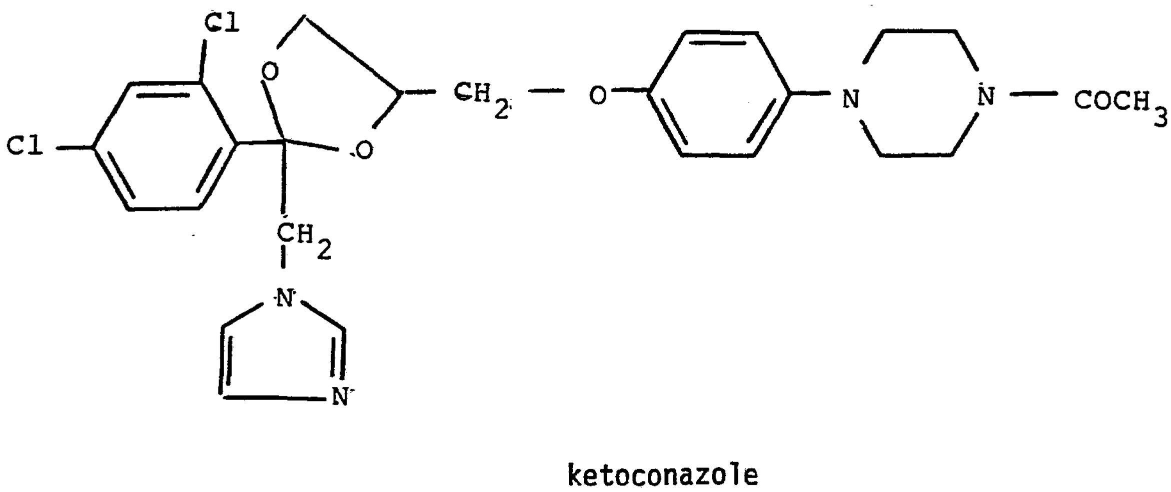

ketoconazole

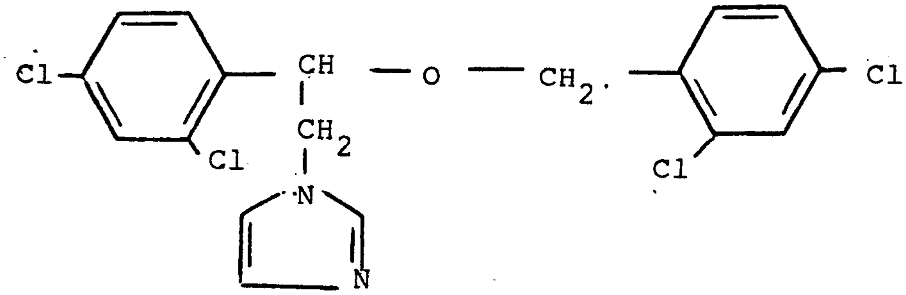

miconazole

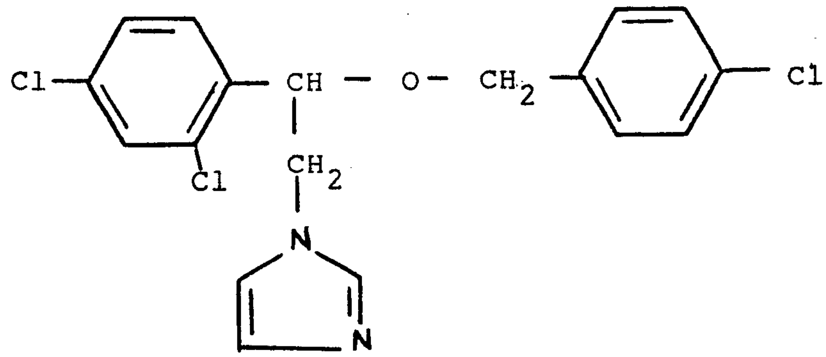

econazole

Figure 1

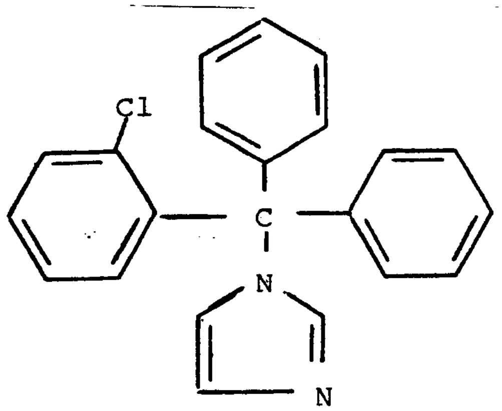

clotrimazole

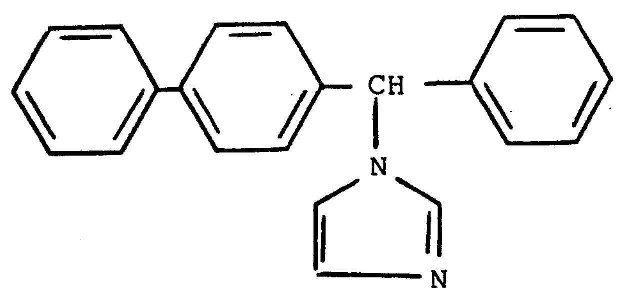

bifonazole

Figure 2

Comme le montrent les figures 1 et 2, tous les dérivés d'imidazole développés par Janssen ont un groupe 1-éthyl imidazole comportant un seul substituant aromatique alors que les composés de Bayer, le clotrimazole et le bifonazole, ont l'un et l'autre, un groupe 1-méthyl imidazole comportant plusieurs substituants aromatiques.

Les auteurs de la présente invention ont choisi une voie intermédiaire en prenant comme base de développement d'une nouvelle série de dérivés d'imidazole, le composé représenté par la figure 3. Ce composé comporte le group 1-éthyl imidazole du miconazole et de l'éconazole et la substitution polyaromatique caractéristique des composés de Bayer.

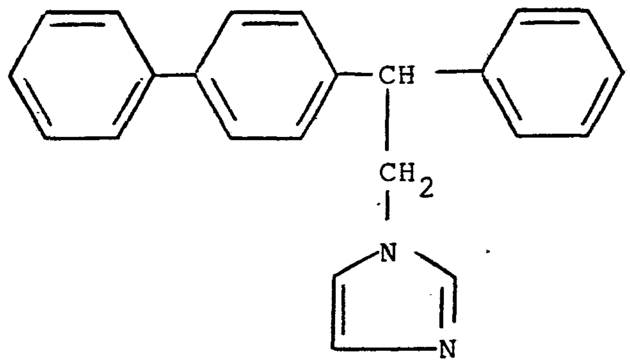

Figure 3

La présente invention concerne la synthèse et les propriétés antifongiques de dérivés de 1-(2,2-diphenyl-ethyl)-1H-imidazole représentés par la figure 4 :

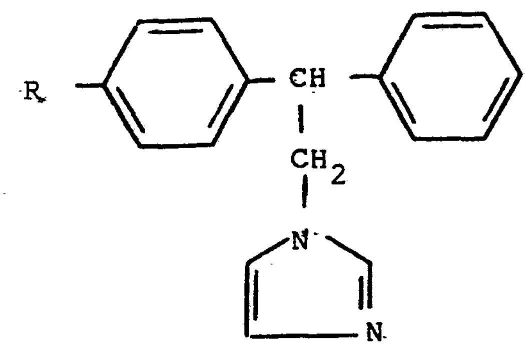

Figure 4

dans laquelle R est un atome d'halogène.

Ces composés ont été découverts par les auteurs de l'invention lors de la mise au point d'un programme de synthèse de nouveaux imidazoles dérivés du composé représenté par la figure 3 par remplacement du phényle du groupe biphényle par un autre substituant tel que halogène, nitro, amino, etc.

La présente invention concerne également les compositions pharmaceutiquement acceptables des composés de la figure 4, qui peuvent être administrées par application topique sur la peau, par voie rectale, intravaginale ou par injection intraveineuse.

Ces compositions pharmaceutiques sont réalisées par mélange du composé actif, éventuellement sous forme de l'un des ses sels pharmaceutiquement acceptables, avec un excipient solide, semi-solide ou liquide.

Le principe actif représente en général, 0,1 à 99 % en poids de la composition pour l'administration intravaginale et 1 à 10 % pour les compositions à usage topique.

La préparation des compositions pharmaceutiques sous forme de doses unitaires pour l'administration

intravaginale est réalisée de la façon suivante : le principe actif est mélangé avec un excipient solide pulvérulent, par exemple : lactose, saccharose, sorbitol, etc..., un amidon tel que amidon de maïs, amylopectine, agar, etc, dérivé de cellulose, polyvinylpyrrolidone, gélatine et, éventuellement, avec un lubrifiant tel que stéarate de magnésium ou de calcium, Carbowax ou autres cires de polyéthylèneglycol. Les comprimés et ovules à usage intravaginal sont ensuite obtenus par compression du mélange.

Les formes pharmaceutiques destinées à l'application topique sur la peau, telles que crème, pommade, gel, solution, poudre, aerosol, sont préparées par mélange du principe actif avec, selon le cas, un constituant de base des crèmes tel que émulsion huile/eau, mélange de cires telles que lanoline, vaseline ou plastibase, solution hydroalcoolique, talc, kaolin ou autres poudres, ou mélange de solvants et d'agents de propulsion tels que Freon 112 ou 14.

Pour l'utilisation en application sur les yeux ou les muqueuses, les compositions peuvent être présentées sous forme de solutions du principe actif dans l'eau salée contenant éventuellement des agents de conservation et/ou des substances tampon.

Pour l'administration parentérale, on peut préparer des solutions aqueuses des composés de l'invention ou de leurs sels d'addition avec des acides tels que les acides chlorhydrique, nitrique, sulfurique, phosphorique, paratoluène sulfonique ou d'autres acides minéraux ou organiques.
Ces solutions contiennent le principe actif, de préférence à la concentration de 0,1 à 2 % et éventuellement un agent stabilisant et/ou des substances tampon.
Les doses unitaires peuvent être présentées sous forme d'ampoules ou de flacons.

Les doses de principe actif administrées peuvent varier dans une certaine mesure dépendant de différents facteurs tels que la voie d'administration ou la gravité de l'infection fongique. Les doses convenant à une application intravaginale sont de l'ordre de 50 à 100 mg, une à trois fois par jour.
Pour l'usage topique, on utilise de préférence des compositions contenant 1 à 5 % de principe actif, appliquées 1 à 3 fois par jour.

Les composés de l'invention peuvent être préparés par différentes méthodes représentées dans les schémas 1-3.

SCHEMA No. 1

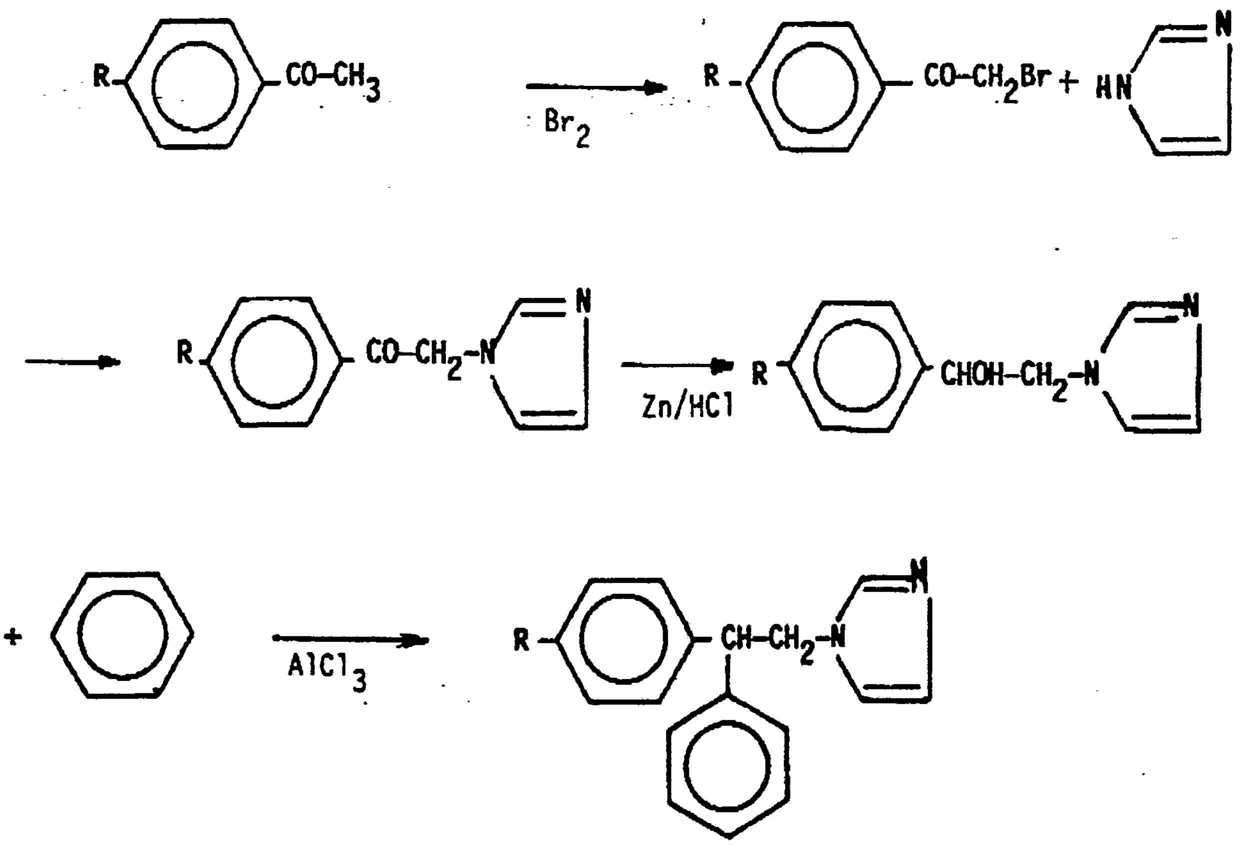


SCHEMA No 2

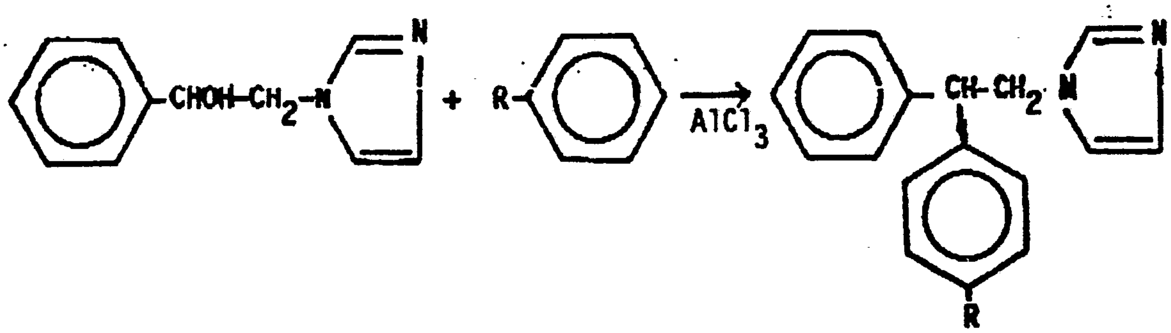

SCHEMA No 3

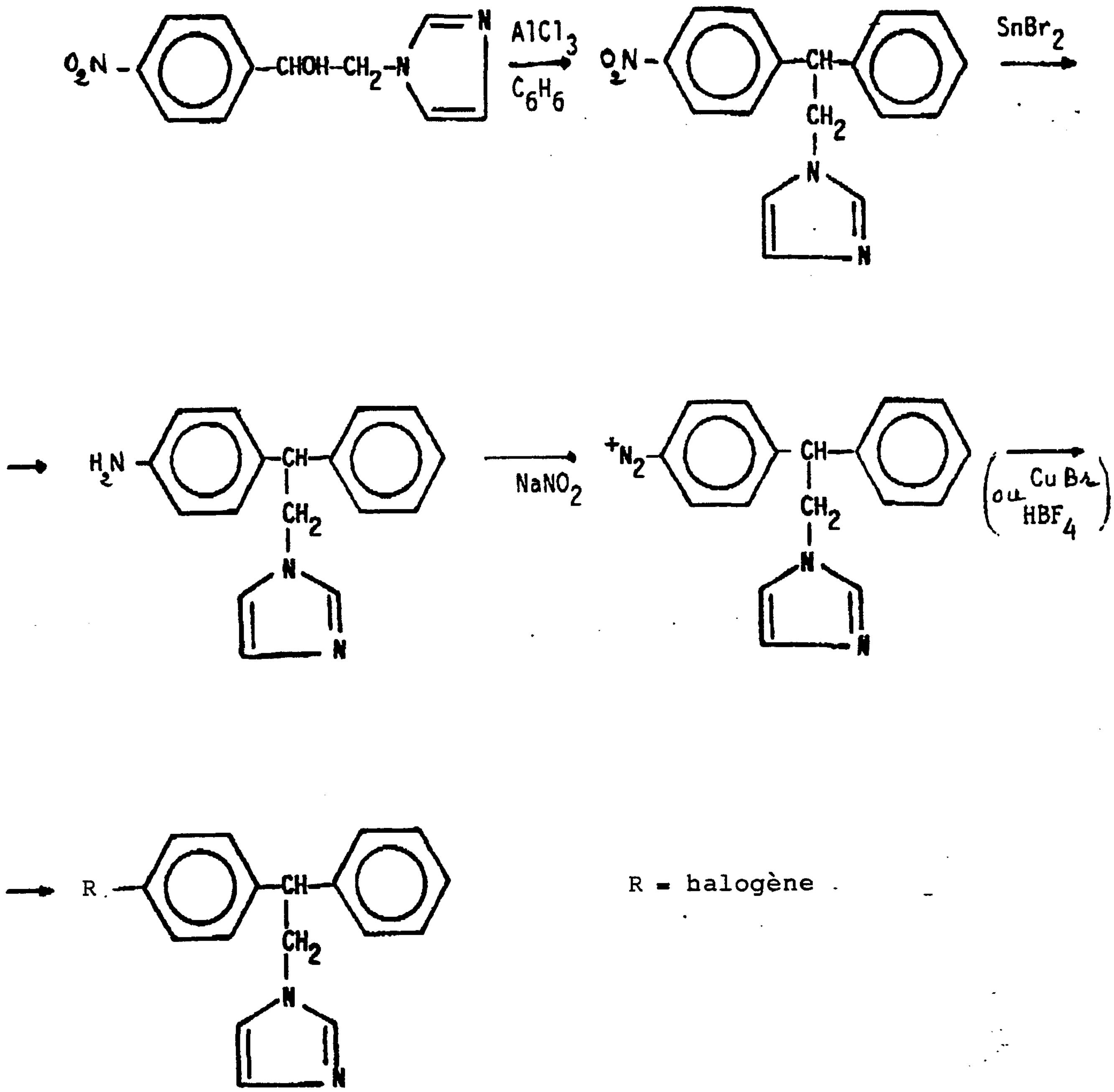


R = halogène .

Selon la méthode 1, la matière première est leα-(p-halophényl)-1H-imidazole-1-ethanol (qui peut être préparé à partir de l'α-bromo ou α-chloro p-halo acétophénone correspondante par réaction avec l'imidazole puis réduction du groupe cétone en groupe alcool) qui réagit avec le benzène selon la méthode de Friedel et Crafts, en présence d'un catalyseur approprié tel le trichlorure d'aluminium anhydre ou le chlorure de zinc.

Selon la méthode 2, la matière première est le α-phényl-1H-imidazole-1-éthanol qui réagit avec un halobenzène par réaction de Friedel et Crafts comme dans la méthode 1.

Selon la méthode 3, la matière première est leα-(p-nitrophenyl)-1H-imidazole-1-éthanol (obtenu par exemple, à partir de la p-nitroacétophénone correspondante par halogénation en alpha, puis réaction avec l'imidazole et réduction du groupe cétone en groupe alcool) qui réagit avec le benzène selon la méthode de Friedel et Crafts, en présence d'un catalyseur approprié, pour donner le 1-[2-(4-nitrophényl)-2-phényléthyl]-1H-imidazole qui est réduit en 1-[2-(4-aminophényl)-2-phényléthyl]-1H-imidazole. Ce dérivé aminé est ensuite transformé en dérivé halogéné par traitement au nitrite de sodium, selon la méthode classique de

diazotation, puis réaction du composé obtenu avec un agent d'halogénation approprié (CuBr, $HBF_4$).

Exemple 1 : 1-[2-(4-bromophényl)-2-phényléthyl]-1H-imidazole.

Méthode 1 :

On a mis en suspension dans un ballon, en chauffant et agitant, 13,3 g (0,1 mol.) de trichlorure d'aluminium anhydre dans 25 ml de benzène sec et 25 ml de 1,2-dichloroéthane.

On a refroidi le mélange à -10°C puis ajouté lentement 13,3 g (0,05 mol.) de α-(4-bromophényl)-1H-imidazole-1-éthanol. A la fin de l'addition, on a laissé la température remonter jusqu'à la température ambiante, en agitant pendant deux heures. Le mélange a ensuite été hyrolysé par 250 ml d'eau froide. On a obtenu une phase benzénique, une phase aqueuse et une couche huileuse insoluble dans les solvants aqueux et organiques. Les deux phases organiques ont été prélevées, lavées avec 100 ml de soude (2N) et extraites deux fois au chloroforme. La phase chloroformique a été séchée et évaporée sous vide. On a obtenu 7,7 g d'un produit huileux non cristallisable.

Ce produit s'est révélé soluble dans le benzène, l'éthanol, l'acétone et le chloroforme et insoluble dans l'eau, le n-hexane, l'éther isopropylique et l'éther éthylique.

La phase aqueuse rendue très alcaline par addition de soude (2N) a fourni un peu de produit supplémentaire.

On a obtenu 13 g de produit (rendement = 79.5 %).

IR : 3090 $cm^{-1}$, 3020 $cm^{-1}$, 3000 $cm^{-1}$, 2900 $cm^{-1}$, 1470 $cm^{-1}$, 1210 $cm^{-1}$, 1000 $cm^{-1}$ et 800 $cm^{-1}$

CCM : silica gel Kieselgel 254 60 F sur aluminium.

solvant : $CHCl_3$ -EtOH (30/70) Rf = 0,68

benzène - AcOEt (4/1) Rf = 0

RMN ($CDCl_3$) 200 MHz :

4,2 - 4,4 ppm (t, 1H, -CH-)

4,5 - 4,6 ppm (d, 2H, $CH_2$)

6,6 - 7,5 ppm (m, 9H aromatiques + 3H de l'imidazole)

CLG : colonne de verre, 1,5 x 4 mm (D.I.)

SE-30 à 5 % sur chromosorb W-60/80 mesh.

Température :

colonne : 260°C

injecteur : 280°C

détecteur : 290°C

k' = 7,76

Préparation du p-toluène sulfonate de 1-[2-(4-bromophényl)-2-phényléthyl]-1H-imidazole.

On a dissous 2 g (0,006 mol.) de base dans 25 ml d'acétone sèche, puis, en agitant vigoureusement, on a versé la solution obtenue, dans une solution de 1,14 g (0,006 mol) d'acide p-toluènesulfonique dans 15 ml d'acétone sèche. Par addition de 20 ml d'éther sec, on a obtenu un précipité blanc qui a été filtré.

P.F. = 123-125°C

IR : 3120 $cm^{-1}$, 3040 $cm^{-1}$, 3000 $cm^{-1}$, 1560 $cm^{-1}$, 1530 $cm^{-1}$, 1200 $cm^{-1}$, 1000 $cm^{-1}$, 800 $cm^{-1}$.

RMN (DMSO) 200 MHz

2,2 ppm (s, 3H, $-CH_3$)

5,1 - 5,3 ppm (t,1H, -CH-)

5,4 - 5,5 ppm (d, 2H,$-CH_2$)

7 - 7,2 ppm (d, 2H, 2CH-de l'imidazole)

7,2 - 7,8 ppm (m, 13H aromatiques)

8,9 - 9 ppm (s, 1H, CH-de l'imidazole).

Préparation du nitrate de 1-[2-(4-bromophényl)-2-phényléthyl]-1H-imidazole.

On a dissous 4 g (0,0122 mol.) de base dans 10 ml d'acétone sèche puis ajouté une solution de 0,92 ml d'acide nitrique (13,28 N) dans 5 ml d'acétone sèche, en agitant vigoureusement. Par addition de 25 ml d'éther sec, on a obtenu un précipité blanc qui a été filtré.

Rendement : 4 g (83 %)

P.F. = 155-156°C

I.R = 3150 $cm^{-1}$, 3075 $cm^{-1}$, 1560 $cm^{-1}$, 1550 $cm^{-1}$ et 1480 $cm^{-1}$.

RMN (DMSO) 60 MHz :

4,7 - 5,2 ppm (m, 3H, $CH-CH_2$)

7,1 - 7,9 ppm (m, 11H, 9H aromatiques + 2H de l'imidazole).

9,1 ppm (s, 1H, CH de l'imidazole)

Préparation du sulfate de 1-[2-(4-bromophényl)-2-phényléthyl]-1H-imidazole.

On a dissous 5 g (0,015 mol.) de base dans 25 ml d'acétone sèche puis ajouté une solution de 0,4 ml d'acide sulfurique (37,5 N) dans 10 ml d'acétone sèche, en agitant vigoureusement.

On a obtenu un précipité blanc qui a été filtré.

Rendement : 4 g (62 %)

P.F. = 207-208°C

I.R = 3120 $cm^{-1}$, 3060 $cm^{-1}$, 1575 $cm^{-1}$, 1550 $cm^{-1}$ et 1490 $cm^{-1}$.

RMN (DMSO) 60 MHz :

4,7 - 5,2 ppm (m, 3H, $CH-CH_2$)

7,1 - 7,7 ppm (m, 11H, 9H aromatiques + 2H de l'imidazole).

8,7 ppm (s, 1H, CH de l'imidazole).

Méthode 2 :

On a mis en suspension dans un ballon, en chauffant et agitant, 13,3 g (0,1 mol.) de trichlorure d'aluminium anhydre dans 25 ml de 1,2-dichloroéthane et 7,9 g (0,05 mol) de bromobenzène.

On a refroidi le mélange à -10°C puis ajouté lentement 9,4 g (0,05 mol.) de α-phényl-1H-imidazole-1-éthanol. A la fin de l'addition, on a laissé la température remonter jusqu'à la température ambiante, en agitant pendant 2 heures. Le mélange a été hydrolysé et les différentes phases isolées de la même façon que dans la méthode 1.

Rendement : 6 g (37 %)

Le produit huileux obtenu a les mêmes propriétés physico-chimiques que le produit obtenu par la méthode 1.

Méthode 3.

3a-Préparation du 1-[2-(4-nitrophényl)-2-phényl-éthyl]-1H-imidazole.

Dans un ballon équipé d'un agitateur magnétique, on a introduit 4,66 g (0,02 mol) de α-(p-nitro-phényl)-1H-imidazole-1-éthanol, 10 ml de benzène sec et 10 ml de 1,2-dichloro éthane.

On a refroidi le mélange à -10°C à l'aide d'un mélange glace-sel, puis ajouté, en agitant vigoureusement, 5,34 g (0,04 mol) de trichlorure d'aluminium anhydre.

On a laissé la température remonter lentement jusqu'à la température ambiante puis agité le mélange pendant une demi-heure.

On a ensuite chauffé à 80°C pendant une heure.

Après avoir refroidi et hydrolysé le mélange par de l'eau froide, on a obtenu un produit huileux qui a été lavé avec 50 ml de soude (2N) et extrait au chloroforme. Le solvant à été séché et éliminé sous vide.

Rendement : 1,7 g (30 %)

IR : 3040 $cm^{-1}$, 2940 $cm^{-1}$, 2860 $cm^{-1}$, 1600 $cm^{-1}$, 1500 $cm^{-1}$, 1450 $cm^{-1}$.

3b-Préparation du 1-[2-(4-bromophényl)-2-phényl-éthyl]-1H-imidazole.

Dans un ballon muni d'un agitateur magnétique et d'un système de chauffage à reflux, on a préparé une solution de bromure stanneux ($SnBr_2$) par chauffage pendant 2 heures, d'un mélange de 2 g d'étain et de 11 g d'acide bromhydrique à 45 %.

On a refroidi à 40°C puis ajouté, en agitant vigoureusement, 1,5 g de 1-[2-(4-nitrophényl)-2-phényléthyl]-1H-imidazole. Au cours de l'addition, la température s'est élevée à 100°C.

Le mélange a ensuite été chauffé au reflux pendant une heure, puis refroidi à 0°C et traité par une solution de 0,4 g de nitrite de sodium dans 2 ml d'eau. Ce mélange a ensuite été versé sur une solution chaude de bromure de cuivre préparée à partir d'une solution de 14,3 g de sulfate de cuivre et 0,7 g de bromure de sodium dans 4 ml d'eau à laquelle a été ajoutée une solution de 0,31 g de métabisulfite de sodium et 0,24 g de soude dans 2 ml d'eau.

On a ensuite ajouté 1,6 ml d'acide bromhydrique à 46 % et abandonné le mélange à la température ambiante pendant 24 heures.

Le mélange a été neutralisé par de la soude (2N) puis extrait au chloroforme.

Le produit huileux obtenu a les mêmes propriétés physico-chimiques que le produit obtenu par la méthode 1.

Exemple 2 : 1-[2-(4-fluorophényl)-2-phényl éthyl]-1H-imidazole.

Méthode 1.

Dans un ballon muni d'un agitateur magnétique, on a mis en suspension 13,3 g (0,1 mol) de trichlorure d'aluminium dans 25 ml de benzène sec et 25 ml de 1,2-dichloroéthane.

On a refroidi à -10°C, puis ajouté lentement 10,3 g (0,05 mol) de α-(p-fluorophényl)-1H-imidazole-1-éthanol. A la fin de l'addition, on a laissé remonter la température jusqu'à la température ambiante puis agité le mélange pendant deux heures.

Le mélange a ensuite été hydrolysé par 250 ml d'eau froide puis la phase organique a été séparée, lavée avec 100 ml de soude (2N) et extraite deux fois au chloroforme.

La phase chloroformique a été séchée et évaporée sous vide. On a obtenu un produit huileux non cristallisable.

Ce produit était soluble dans le benzène, l'éthanol, l'acétone et le chloroforme et insoluble dans l'eau, le n-hexane, l'éther isopropylique et l'éther éthylique.

La phase aqueuse fortement alcalinisée par de la soude (2N) a fourni un peu de produit supplémentaire.

Rendement : 4,6 g (34,5 %).

IR : 3090 $cm^{-1}$, 3020 $cm^{-1}$, 3000 $cm^{-1}$, 2900 $cm^{-1}$, 1490 $cm^{-1}$.

CCM : Silica gel Kieselgel 254 60 F sur aluminium.

solvant : $CHCl_3$ -EtOH (30/70) Rf = 0,69

benzène - AcOEt (4/1) Rf = 0

RMN ($CDCl_3$) 60 MHz :

4,1 - 4,7 ppm (m, 3H, CH-$CH_2$)

6,6 - 7,5 ppm (m, 11H, 9H aromatiques + 3H de l'imidazole).

CLG : colonne : verre, 1,5 x 4 mm (D.I.)

SE-30 à 5 % sur chromosorb W-60/80 mesh.

Températures :

colonne : 260°C

injecteur : 280°C

détecteur : 290°C

k' = 3,02

Préparation du p-toluènesulfonate de 1-[2-(4-fluorophényl)-2-phényléthyl]-1H-imidazole.

On a dissous 2 g (0,0076 mol.) de base dans 25 ml d'acétone sèche puis, en agitant vigoureusement, on a versé la solution obtenue dans une solution de 1,44 g (0,0076 mol.) d'acide p-toluènesulfonique dans 15 ml d'acétone sèche. Par addition de 20 ml d'éther sec, on a obtenu un précipité blanc qui a été filtré.

P.F. = 106-110°C

IR : 3120 $cm^{-1}$, 3040 $cm^{-1}$, 3000 $cm^{-1}$, 1560 $cm^{-1}$, 1530 $cm^{-1}$.
RMN (DMSO) 60 MHz
2,2 ppm (s, 3H, $-CH_3$)
4,7 - 5,1 ppm (m, 3H, $CH-CH_2$)
6,9 - 7,8 ppm (m, 15H, 13H aromatiques + 2H de l'imidazole).
9 ppm (m, 1H, CH de l'imidazole.)

Préparation du sulfate de 1-[2-(4-fluorophényl)-2-phényléthyl]-1H-imidazole.

On a dissous 4 g (0,015 mol.) de base dans 25 ml d'acétone sèche puis, en agitant vigoureusement, on a ajouté une solution de 0,4 ml d'acide sulfurique (37,5 N) dans 10 ml d'acétone sèche.
Le précipité blanc qui s'est formé, a été filtré.
Rendement : 3 g (55 %)
P.F. = 170-174°C
IR = 3140 $cm^{-1}$, 3060 $cm^{-1}$, 1575 $cm^{-1}$, 1510 $cm^{-1}$, 1220 $cm^{-1}$
RMN (DMSO) 60 MHz :
4,7 - 5,2 ppm (m, 3H, $CH-CH_2$)
7,1 - 7,8 ppm (m,11H, 9H aromatiques + 2H de l'imidazole).
8,6 ppm (s, 1H, CH de l'imidazole).

Méthode 2.

On a mis en suspension dans un ballon, en chauffant et agitant, 13,3 g (0,1 mol) de trichlorure d'aluminium anhydre dans 25 ml de 1,2-dichloroéthane et 4,8 g (0,05 mol) de fluorobenzène.
On a refroidi à -10°C puis ajouté lentement 9,4 g (0,04 mol.) de α-phényl-1H-imidazole-1-éthanol.
A la fin de l'addition, on a laissé remonter la température jusqu'à la température ambiante puis agité pendant 2 heures.
Le mélange a été hydrolysé et ses constituants ont été séparés de la même façon que dans la méthode 1.
Rendement : 3,6 g (27 %)
Le produit huileux obtenu a les mêmes propriétés physico-chimiques que le produit obtenu par le méthode 1.

Méthode 3.

3a-Préparation du 1-[2-(4-nitrophényl)-2-phényléthyl]-1H-imidazole.

Ce composé est préparé selon la méthode décrite dans l'exemple 1, méthode 3 (étape 3a).

3b-Préparation du 1-[2-(4-aminophényl)-2-phényléthyl]-1H-imidazole.

Dans un ballon muni d'un agitateur et d'un réfrigérant à reflux, on a introduit 2,93 g (0,01 mol.) de 1-[2-(4-nitrophényl)-2-phényléthyl]-1H-imidazole dans 25 ml d'éthanol à 95 % et 2 ml d'une solution de soude à 20 %. On a agité vigoureusement le mélange puis chauffé au bain de vapeur jusqu'à légère ébullition. Le bain de vapeur à ensuite été enlevé et 5 g de poudre de zinc ont été ajoutés assez lentement pour maintenir le mélange à l'ébullition. A la fin de l'addition de zinc, le mélange a été chauffé au reflux sous agitation continue, pendant une heure.
Le mélange a été filtré à chaud puis le filtrat concentré sous pression réduite.
On a refroidi puis ajouté 25 ml d'eau et extrait le produit au chloroforme.
La phase organique a été séchée et évaporée sous vide. On a obtenu un produit huileux.
Rendement : 2,1 g (80 %).

3c-Préparation du 1-[2-(4-fluorophényl)-2-phényléthyl]-1H-imidazole.

On a agité un mélange de 20 ml d'acide chlorhydrique (2N) et de 2,1 g (0,008 mol.) de 1-[2-(4-aminophényl)-2-phényléthyl]-1H-imidazole, en refroidissant à l'aide d'un mélange glace-sel. On a alors ajouté lentement une solution de 0,6 g de nitrite de sodium dans 5 ml d'eau, en maintenant la température en dessous de 7°C pendant la diazotation.

On a d'autre part préparé de l'acide fluoborique par addition de 0,5 g d'acide borique à 0,7 g d'acide fluorhydrique à 60 %. Cette opération a été réalisée dans un ballon enduit de cire, agité et maintenu froid à l'aide d'un bain glace-eau.

La solution d'acide fluoborique refroidie par de la glace a ensuite été versée dans la solution du diazonium refroidie à une température inférieure à 0°C. On a agité pendant 30 minutes, puis filtré le solide formé qui a été lavé successivement à l'eau froide, à l'alcool méthylique et à l'éther, en essorant le mieux possible après chaque lavage.

Le fluoborate ensuite été séché sur acide sulfurique concentré, dans un dessicateur sous vide. Le dérivé fluoré a été obtenu par décomposition thermique du fluoborate dans un ballon à distiller relié par un condenseur à un autre ballon équipé d'un tube de dégagement pour évacuer les vapeurs de fluorure de bore.

On a chauffé doucement jusqu'au début de la décomposition puis laissé la réaction évoluer spontanément, en chauffant de temps en temps pour maintenir la réaction.

On a finalement chauffé fortement le ballon pour terminer le processus de décomposition.

Le dérivé fluoré a été extrait au chloroforme, séché et évaporé sous vide. On a obtenu un produit huileux.

Rendement : 1,1 g (52 %)

Le produit obtenu a les mêmes propriétés physico-chimiques que le produit obtenu par la méthode 1.

Les propriétés antifongiques des composés de l'invention ont été étudiées vis à vis de souches de champignons sélectionnées:Candida, Microsporum et dermatophytes.

Cette étude a été faite en comparaison avec le bifonazole et le ketoconazole, deux médicaments antifongiques commercialisés récemment. On a utilisé des disques de papier filtre (diam. = 1/4) sur lesquels on a déposé 50 µl d'une solution à 1 mg/ml dans l'ëthanol de composé de l'invention ou de composé de référence (soit 50 µg de composé).

Les disques sont séchés puis placés dans des boîtes de Petri contenant 10 ml d'agar Sabouraud ensemencé par une suspension de cellules ou de spores provenant des souches sélectionnées.

Le diamètre des zones d'inhibition a été mesuré au bout de 24 heures dans le cas de l'espacé Candida et au bout de 3 à 4 jours pour tous les autres champignons.

Le tableau 1 montre les résultats de cette première série d'essais. Les composés de l'invention sont aussi actifs que le kétoconazole vis à vis de l'espèce Candida albicans mais plus actifs vis à vis des autres espèces Candida (pseudotropicalis, utilis...) et vis à vis des dermatophytes.

Les composés de l'invention se sont révélés beaucoup plus actifs que le bifonazole vis à vis de toutes les souches étudiées.

Tableau I . Activité antifongique des composés de l'invention comparée à celle du bifonazole (BFZ) et du kétoconazole (KTZ) vis à vis de souches de champignons sélectionnées (50 µg/disque).

| Microorganisme | Composé de l'invention Br | Composé de l'invention F | Composé de référence BFZ | Composé de référence KTZ |
|---|---|---|---|---|
| Candida albicans | ++ | ++ | + | +++ |
| Candida pseudotropicalis | +++ | +++ | ++ | +++ |
| Candida utilis | +++ | +++ | ++ | +++ |
| Candida parakrusei | +++ | ++ | ++ | +++ |
| Aspergillus niger | ++++ | +++ | + | ++ |
| Aspergillus flavus | +++ | +++ | ++ | +++ |
| Cladosporium herbarum | +++ | ++ | ++ | ++ |
| Nocardia corallina | ++ | ++ | + | ++ |
| Trichosporum cutaneum | ++++ | ++++ | +++ | +++ |
| Saccharomyces cerevisae | +++ | ++ | ++ | +++ |
| Trichophyton mentagrophytes | ++++ | ++++ | +++ | +++ |

++++ diamètre de la zone d'inhibition $\geqslant$ 50 mm

+++ diamètre de la zone d'inhibition : 40-50 mm

++ diamètre de la zone d'inhibition : 20-40 mm

La toxicité aiguë des composés de l'invention a été déterminée par voie orale chez les souris Swiss des deux sexes, par la méthode de Lichfield et Wilcoxon. Des groupes de cinq souris de chaque sexe ont été traités par des doses croissantes du composé étudié et le taux de mortalité a été déterminé au cours des 7 jours suivant l'administration du produit.

On a trouvé les doses léthales ($DL_{50}$ en mg/kg) suivantes :

| | dérivé bromé | dérivé fluoré |
|---|---|---|
| souris mâle | 450 (400-475) | 524 (457-538) |
| souris femelle | 375 (350-400) | 465 (438-502) |

On a également étudié le pouvoir mutagène des composés de l'invention. Cette étude a été réalisée au moyen du test Salmonella-microsome (test de Ames) avec et sans activation par des microsomes hépatiques du rat. Des plaques d'agar ensemencées par des souches de Salmonella typhimurium (Ames)

TA-1535, TA-1537, TA-1538, TA-98 et TA-100 ont été incubées en présence des composés de l'invention à des concentrations croissantes, en présence et en l'absence de microsomes hépatiques provenant de rats traités au préalable par l'Aroclor ou le phénobarbital.

Le pouvoir mutagène a été déterminé au bout de 24 heures d'incubation par comptage du nombre de colonies mutantes.

Jusqu'à des doses de 100 µg/plaque, les composés de l'invention n'ont présenté aucun pouvoir mutagène, aussi bien en présence qu'en l'absence d'activitation microsomique.

Des doses supérieures à 100 µg/plaque se sont révélées toxiques pour les bactéries en raison de l'activité bactéricide.

**Revendications**

1) Nouveaux dérivés d'imidazole, de formule (I) :

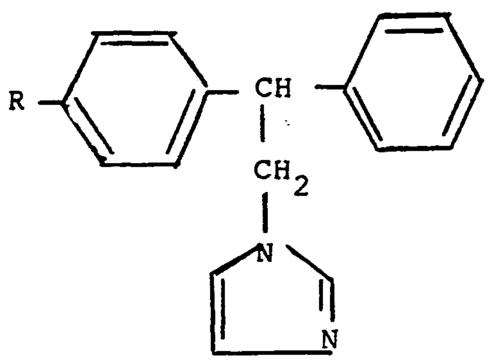

(I)

dans laquelle R représente un atome d'halogène.

2) Selon la revendication 1, les composés de formule (I) dans laquelle R représente un atome de fluor.

3) Selon la revendication 1, les composés de formule (I) dans laquelle R représente un atome de brome.

4) Selon l'une quelconque des revendications 1 à 3, les sels d'addition pharmaceutiquement acceptables des composés de formule (I), tels que notamment nitrate, sulfate, phosphate, p-toluènesulfonate, préparés par réaction du composé de formule (I) avec l'acide minéral ou organique correspondant.

5) Procédé de préparation des composés de formule (I) selon les revendications 1 à 3 qui consiste à traiter un α-(4-halophényl)-1H-imidazole-1-éthanol par le benzène selon la méthode de Friedel et Crafts, en présence d'un catalyseur du type base de Lewis, tel que trichlorure d'aluminium anhydre, chlorure de zinc, acide sulfurique et d'un solvant tel que benzène anhydre, n-hexane, 1,2-dichloroéthane ou tout autre solvant non polaire, à une température comprise entre -10°C et 120°C.

6) Procédé de préparation des composés de formule (I) selon les revendications 1 à 3, qui consiste à traiter 1'α-phényl-1H-imidazole-1-éthanol par un halobenzène selon la méthode de Friedel et Crafts, dans des conditions analogues à celles du procédé de la revendication 5.

7) Procédé de préparation des composés de formule (I) selon les revendications 1 à 3, qui consiste à traiter le 1-[2-(4-nitrophényl)-2-phényléthyl]-1H-imidazole par un agent réducteur (tel que Sn $Br_2$, Zn/NaOH), puis à transformer le 1-[2-(4-aminophényl)-2-phényl-éthyl]-1H-imidazole ainsi obtenu en dérivé halogéné correspondant par diazotation et traitement par un agent d'halogénation.

8) Composés de formule (I) et leurs sels pharmaceutiquement acceptables, selon l'un quelconque des revendications 1 à 4, utilisés comme médicaments.

Revendications pour les états contractants suivants: AT,GR,ES.

1) Procédé de préparation de nouveaux dérivés d'imidazole, de formule (I) :

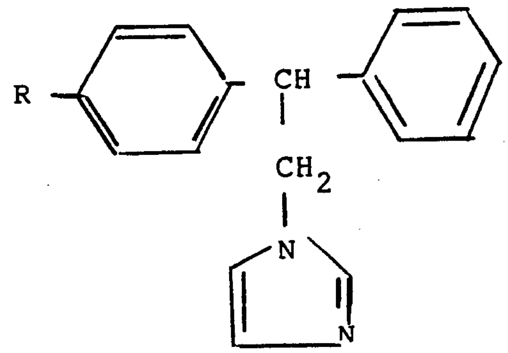

(I)

dans laquelle R représente un atome d'halogène, qui consiste à traiter unα-(4-halophényl)-1H-imidazole-1-éthanol par le benzène selon la méthode de Friedel et Crafts, en présence d'un catalyseur du type base de Lewis, tel que trichlorure d'aluminium anhydre, chlorure de zinc, acide sulfurique et d'un solvant tel que benzène anhydre, n-hexane, 1,2-dichloroéthane ou tout autre solvant non polaire, à une température comprise entre -10°C et 120°C.

2) Procédé de préparation des composés de formule (I) qui consite à traiter 1'α-phényl-1H-imidazole-1-éthanol par un halobenzène selon la méthode de Friedel et Crafts, dans des conditions analogues à celles du procédé de la revendication 1.

3) Procédé de préparation des composés de formule (I) qui consiste à traiter le 1-[2-(4-nitrophényl)-2-phényléthyl]-1H-imidazole par un agent réducteur (tel que Sn $Br_2$, Zn/NaOH), puis à transformer le 1-[2-(4-aminophényl)-2-phényl-éthyl]-1H-imidazole ainsi obtenu en dérivé halogéné correspondant par diazotation et traitement par un agent d'halogénation.

4) Procédé de préparation selon l'une quelconque des revendications 1 à 3, des composés de formule (I) dans laquelle R représente un atome de fluor.

5) Procédé de préparation, selon l'une quelconque des revendications 1 à 3, des composés de formule (I) dans laquelle R représente un atome de brome.

6) Procédé de préparation des sels d'addition pharmaceutiquement acceptables des composés de formule (I) obtenus selon l'une quelconque des revendications 1 à 6, tels que notamment nitrate, sulfate, phosphate, p-toluènesulfonate, qui consiste à faire réagir les composés de formule (I) avec l'acide minéral ou organique correspondant.

Office européen des brevets

**RAPPORT DE RECHERCHE EUROPEENNE**

Numéro de la demande

EP 87 40 0193

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | US-A-4 115 578 (G.A. MILLER et al.)<br>* table I, exemples 93, 17, 18, 24, 25 en combinaison avec exemple 93 * | 1-3 | C 07 D 233/56<br>A 61 K 31/415 |
| Y | * colonne 1, ligne 10 - colonne 2, ligne 3 *<br>--- | 8 | |
| X | EP-A-0 068 144 (HOECHST AG)<br>* page 1, ligne 12 - page 3, ligne 35; page 6, lignes 9-17; exemple 37 en combinaison avec exemples 11, 7, 8 * | 1,3-6, 8 | |
| Y | * page 1, lignes 12-17 *<br>--- | 8 | **DOMAINES TECHNIQUES RECHERCHES (Int. Cl.4)**<br>C 07 D 233/00<br>A 61 K 31/00<br>C 07 D 521/00 |
| A | STREITWIESER & HEATHCOCK. INTRODUCTION TO ORGANIC CHEMISTRY, 1981, chapitres 24, 25, 2ème édition, Macmillan Publishing Co., Inc., New York, US;<br>* pages 745, 795, 796 *<br>--- -/- | 7 | |

Le présent rapport de recherche a été établi pour toutes les revendications

| Lieu de la recherche | Date d'achèvement de la recherche | Examinateur |
|---|---|---|
| BERLIN | 07-09-1987 | VAN AMSTERDAM L.J.P. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82

Office européen des brevets

**RAPPORT DE RECHERCHE EUROPEENNE**

Numéro de la demande

EP 87 40 0193



## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | CHEMICAL ABSTRACTS, vol. 96, no. 21, 24 mai 1982, page 714, colonne 2, abrégé no. 181282u, Columbus, Ohio, US; & JP - A - 56 164 168 (SUMITOMO CHEMICAL CO. LTD.) 17-12-1981 | 1 | |
| A | EP-A-0 037 049 (BAYER AG) * revendications 1, 9, 10 * | 1,4,8 | |

DOMAINES TECHNIQUES RECHERCHES (Int. Cl.4)

Le présent rapport de recherche a été établi pour toutes les revendications

| Lieu de la recherche | Date d'achèvement de la recherche | Examinateur |
|---|---|---|
| BERLIN | 07-09-1987 | VAN AMSTERDAM L.J.P. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons
& : membre de la même famille, document correspondant

OEB Form 1503 03 82